# EUROPÄISCHE PATENTSCHRIFT

(11) **EP 2 060 236 B1**
(45) Veröffentlichungstag und Bekanntmachung des Hinweises auf die Patenterteilung: **24.04.2013**
(21) Anmeldenummer: 08169062.0
(22) Anmeldetag: 13.11.2008
(51) Int. Cl.: A61B 17/42

(54) **Medizinisches Instrument zum Manipulieren einer Gebärmutter**
Medical instrument for manipulating the uterus
Instrument médical destiné à la manipulation de l'utérus

(30) Priorität: 16.11.2007 DE 102007056388
(43) Veröffentlichungstag der Anmeldung: 20.05.2009
(73) Patentinhaber: Karl Storz GmbH & Co. KG, 78532 Tuttlingen (DE)
(72) Erfinder: Walter, Christian, 78576 Emmingen (DE); Donnez, Jacques, Prof. Dr., 1150 Brüssel (BE)
(74) Vertreter: Witte, Weller & Partner

(56) Entgegenhaltungen:
- EP-A- 0 400 458
- WO-A-2008/074054
- US-A1- 2003 187 334
- US-A1- 2005 277 948

## Beschreibung

Die Erfindung betrifft ein medizinisches Instrument zum Manipulieren einer Gebärmutter, mit einem stabförmigen Körper, dessen distaler Abschnitt durch die Vagina in die Gebärmutter einführbar ist, wobei der stabförmige Körper ein glockenförmiges Element trägt, das längs des stabförmigen Körpers hin und her bewegbar ist, und das glockenförmige Element längs des stabförmigen Körpers verschiebbar ausgebildet ist.

Ein medizinisches Instrument zum Manipulieren einer Gebärmutter mit diesen konstruktiven Merkmalen ist aus der US 2003/0187334 A1 bekannt.

Solche medizinischen Instrumente, die auch Uterusmanipulatoren genannt werden, dienen unter anderem dazu, bei laparoskopischen Untersuchungen im Unterleib einer weiblichen Patientin oder als Vorbereitung für einen chirurgischen Eingriff den Uterus (Gebärmutter) in eine gewünschte Stellung zu bringen. Dies geschieht z.B. dazu; um eine verdeckte Seite des Uterus untersuchen zu können oder um einen chirurgischen Eingriff, beispielsweise eine Hysterektomie (Entfernung der Gebärmutter), durchzuführen.

Ferner offenbaren auch die US 2005/0277948 A1, die EP 0 400 558 A1 und die WO 2008/074054 A1 ähnliche medizinische Instrumente.

Die US 2005/0277948 A1 zeigt dabei ein medizinisches Instrument zum Anbringen einer Cerclage an dem Gebärmutterhals. Dazu weist diese Vorrichtung zwei trichterförmige Elemente auf, von denen das eine entlang eines inneren Schaftes führbar ist.

Die EP 0 400 458 A1 offenbart eine Vorrichtung, die ein konisches Element aufweist, das am distalen Ende der medizinischen Vorrichtung axial verschiebbar angeordnet ist, und das für einen operativen Vorgang mit seinem durchschnittsgeringeren Ende zuerst in die Gebärmutter eingeführt wird.

Das medizinische Instrument aus der WO 2008/074054 A1 weist eine Kanüle mit einem Außengewinde auf, entlang dem ein entsprechendes glockenförmiges Element axial verschoben werden kann.

Das eingangs genannte medizinische Instrument aus der US 2003/0187334 A1 weist einen geradlinigen Stab auf, der nahezu über seine gesamte Länge mit einem groben Außengewinde versehen ist. Auf dem Außengewinde sitzt ein glockenförmiges Element, das durch Drehen längs des Stabes hin und her bewegbar ist. Durch eine relative Drehbewegung zwischen dem glockenförmigen Element und dem Stab kann die axiale Position des glockenförmigen Elements an dem Stab verändert werden. Bei dieser relativen Drehbewegung kann entweder das glockenförmige Element vom Operateur festgehalten werden, und der Stab wird gedreht, oder der Stab wird vom Operateur festgehalten, und das glockenförmige Element wird gedreht.

Zum Manipulieren der Gebärmutter, wird zunächst das glockenförmige Element an den distalen Abschnitt des Stabes gebracht. Dann wird der Stab mit dem an seinem distalen Abschnitt angeordneten glockenförmigen Element in die Vagina so weit eingeführt, bis das glockenförmige Element den Muttermund der Gebärmutter umschließt. In dieser Position wird das glockenförmige Element von der Vaginawand festgehalten. Dann wird der mit dem groben Gewinde versehene Stab von dem Operateur gedreht, so dass er in die Gebärmutter hineinfährt. Mit dem derart positionierten Instrument kann der Operateur durch Bewegung des Stabes die Gebärmutter manipulieren, um eine Untersuchung oder einen chirurgischen Eingriff durchzuführen.

Bei diesem medizinischen Instrument ist nachteilig, dass die Anderung der axialen Position des glockenförmigen Elements an dem Stab durch eine Drehbewegung zwischen dem glockenförmigen Element und dem Stab erfolgt. Um das glockenförmige Element von einem proximalen Ende zu einem distalen Ende zu bewegen, muss der Operatur ca. 20-25 Mal entweder den Stab oder das glockenförmige Element drehen. Dies ist umständlich, und das Einführen und Positionieren des medizinischen Instruments in dem Körper der Patientin benötigt relativ viel Zeit.

Ein weiterer Nachteil des genannten Instruments besteht darin, dass das glockenförmige Element an dem distalen Abschnitt des Stabes bei dessen Einführen in die Gebärmutter angeordnet ist. Dadurch ist der distale Abschnitt des Stabes für den Operateur nicht sichtbar. Somit kann er die Position des distalen Abschnitts des Stabes, der in die Gebärmutter hineingedreht wird, nicht kontrollieren und somit auch nicht korrigieren. Dies kann dazu führen, dass ein falsch positionierter distaler Abschnitt des Stabes nicht in den Hohlraum in der Gebärmutter hineinfährt, sondern in das Gewebe der Gebärmutter eingetrieben wird, wodurch das empfindliche Gewebe durch das grobe Gewinde verletzt werden kann. Das grobe Gewinde beinhaltet die Gefahr, dass Gewebebereiche daran anhaften und beim Drehen mit verdreht werden, so dass der Gebärmutterhals verdrillt wird.

Der Erfindung liegt daher die Aufgabe zugrunde, ein medizinisches Instrument der eingangs genannten Art dahingehend weiterzuentwickeln, dass das Einführen und das Positionieren des Instruments in dem Körper der Patientin für den Operateur einfach und schnell durchzuführen ist und für die Patientin möglichst atraumatisch verläuft.

Erfindungsgemäß wird diese Aufgabe hinsichtlich des eingangs genannten medizinischen Instruments dadurch gelöst, dass das glockenförmige Element mittels eines Arretiermechanismus in unterschiedlichen axialen Positionen an dem stabförmigen Körper arretierbar ist.

Ein Verschieben des glockenförmigen Elements längs des stabförmigen Körpers ist einfach, rasch und ohne ein Drehen der beiden Bauteile relativ zueinander durchzuführen.

Beim Einführen und Positionieren des derartig ausgebildeten medizinischen Instruments kann zunächst das glockenförmige Element nach proximal zum Ende des stabförmigen Körpers verschoben werden, so dass das glockenförmige Element beim Einführen des distalen Abschnitts des stabförmigen Körpers in die Gebärmutter dem Operateur das Operationsfeld nicht versperrt. Der distale Abschnitt des stabförmigen Körpers kann von dem Operateur durch die Vagina in die Gebärmutter sicher und rasch eingeführt werden, wobei die Position des distalen Abschnitts des stabförmigen Körpers von dem Operateur besser erkannt und korrigiert werden kann. Dadurch ist die Gefahr, dass der distale Abschnitt des stabförmigen Körpers das Gewebe der Gebärmutter verletzt, erheblich herabgesetzt. Das glockenförmige Element kann durch einfaches Verschieben nach distal längs des stabförmigen Körpers in die Vagina so weit eingeführt werden, bis es den Muttermund umschließt. Mit dem derartig positionierten Instrument kann die Gebärmutter von dem Operateur manipuliert werden.

Somit ist das Einführen und Positionieren des erfindungsgemäßen Instruments einfach und schnell durchführbar, was zu einer deutlichen Verkürzung der Operationsdauer beiträgt. Ferner ist dieser Vorgang mit dem derartig ausgebildeten Instrument für die Patientin mit einem erheblich geringeren Verletzungsrisiko durchzuführen.

Der Operateur kann z.B. das glockenförmige Element zum Einführen des stabförmigen Körpers durch die Vagina in die Gebärmutter in einer axialen Position arretieren, in der das glockenförmige Element das Operationsfeld nicht bedeckt. Ferner kann der Operateur, nachdem er das glockenförmige Element arretiert hat, sich nur auf die Einführung des stabförmigen Körpers in die Gebärmutter konzentrieren, ohne dass die Gefahr besteht, dass sich die Position des glockenförmigen Elements an dem Stab ändert. Nach dem Positionieren kann das glockenförmige Element in eine andere axiale Position, z.B. eine mehr distale Position, verschoben und dort wieder arretiert werden.

In einer weiteren Ausgestaltung der Erfindung weist der Arretiermechanismus ein an dem glockenförmigen Element angeordnetes Arretierelement auf, das in den unterschiedlichen axialen Positionen in eine Arretierstelle an dem stabförmigen Körper eingreift.

Diese Maßnahme hat den Vorteil, dass ein derartig ausgebildeter Arretiermechanismus besonders handhabungsfreundlich ist, da das an dem verschiebbaren glockenförmigen Element angeordnete Arretierelement für den Operateur, der das glockenförmige Element längs des stabförmigen Körpers verschiebt, einfach greifbar und damit einfach betätigbar ist. Dies trägt ebenfalls zur schnellen und sicheren Handhabung des erfindungsgemäßen Instruments bei.

In einer weiteren Ausgestaltung der Erfindung ist eine erste Arretierstelle an einem proximalen Ende des stabförmigen Körpers angeordnet.

Hierbei besteht der Vorteil, dass das glockenförmige Element beim Einführen des distalen Abschnitts des stabfönnigen Körpers durch die Vagina in die Gebärmutter zunächst an dem proximalen Ende des stabförmigen Körpers arretiert werden kann. In dieser axialen Position stört das glockenförmige Element dieses Einführen am wenigsten.

In einer weiteren Ausgestaltung der Erfindung ist eine zweite Arretierstelle an dem distalen Abschnitt des stabförmigen Körpers angeordnet.

Diese Maßnahme hat den Vorteil, dass das glockenförmige Element in einer Position, in der es den Muttermund der Gebärmutter bereits umschließt, arretiert werden kann. Dies ist für den Operateur vorteilhaft, weil er das glockenförmige Element in dieser Position nicht selbst halten muss. Somit kann er mit dem so in dem Körper der Patientin positionierten Instrument die Gebärmutter durch Bewegen des stabförmigen Körpers manipulieren, ohne dass die Gefahr besteht, dass das glockenförmige Element seine Position im Inneren des Körpers der Patientin ändert. Dies ist schonend für die Patientin, denn das in der Vagina befindliche glockenförmige Element muss nicht durch zusätzliche Gerätschaften in dieser Position fixiert werden, was für die Patientin unangenehm wäre.

In einer weiteren Ausgestaltung der Erfindung ist das Arretierelement in den Arretierstellen selbst einrastend.

Hierbei ist von Vorteil, dass das Arretierelement in die Arretierstelle automatisch eingreift, was beispielsweise durch den typischen Arretierungsklick bemerkt werden kann, so dass dadurch die Handhabungsperson erkennt, dass die Arretierstellung erreicht worden ist. Besonders erleichtert ist das Verschieben nach distal, wenn diese Arretierstelle der maximalen distalen Vorschubposition entspricht. Der Operateur muss das glockenförmige Element nur so lange nach distal verschieben, bis die Arretierung einrastet. Dadurch ist auch ausgeschlossen, dass das glockenförmige Element versehentlich zu weit nach distal verschoben wird.

In einer weiteren Ausgestaltung der Erfindung sind die Arretierstellen als Aussparungen in dem stabförmigen Körper ausgebildet.

Diese Maßnahme hat den Vorteil, dass die Arretierstellen durch einen einfachen Bearbeitungsvorgang an dem stabförmigen Körper ausgebildet werden können. In diese Arretierstelle kann das selbst einrastende Arretierelement derart einrasten, dass der Operateur, der den stabförmigen Körper hält, dies taktil erfassen kann.

In einer weiteren Ausgestaltung der Erfindung ist das Arretierelement als federbelastete Raste ausgebildet.

Dadurch wird ein konstruktiv einfaches Arretierelement geschaffen, das über die Federbelastung in die Arretierstellen hineingedrückt wird, so dass eine ausreichende Verrastung bewerkstelligt werden kann. Darüber hinaus hat das als federbelastete Raste ausgebildete Arretierelement den Vorteil, dass mit einem derartigen Element bei entsprechender Federkraft das Einrasten vom Operateur deutlich gehört und gespürt werden kann, insbesondere, wenn das Rastelement und der stabförmige Körper, in dem die Arretierstellen angeordnet sind, metallische Teile sind.

In einer weiteren Ausgestaltung der Erfindung weist der Arretiermechanismus ein Betätigungselement zum Entsperren des Arretierelements auf, das an dem glockenförmigen Element angeordnet ist, das vorzugsweise als Druckknopf ausgebildet ist.

Diese Maßnahme hat den Vorteil, dass durch einfaches Drücken des Druckknopfs das Arretierelement mit der jeweiligen Arretierstelle außer Eingriff bringbar ist.

In einer weiteren Ausgestaltung der Erfindung weist das glockenförmige Element distalseitig einen zylindrischen Abschnitt auf.

Diese Maßnahme hat den Vorteil, dass der zylindrische Abschnitt des glockenförmigen Elements der anatomischen Form der Vagina nahe kommt, und die Einführung des medizinischen Instruments durch die Vagina einfach durchführbar ist. Beim Anlegen an den Muttermund wird dieser in dem zylindrischen Abschnitt lagegerecht positioniert.

In einer weiteren Ausgestaltung der Erfindung ist das glockenförmige Element an seinem proximalen Ende geschlossen.

Das glockenförmige Element kann an die Gebärmutter nach proximal gasdicht abschließend angelegt werden. Dadurch ist es möglich, den Innenraum der Gebärmutter zur Vergrößerung des Operationsfeldes durch Einführen von Gasen zu erweitern.

In einer weiteren Ausgestaltung der Erfindung sind an dem glockenförmigen Element nach distal vorspringende Fixierelemente angeordnet.

Hierbei ist von Vorteil, dass die nach distal vorspringenden Fixierelemente das glockenförmige Element an dem Muttermund fixieren. Dadurch wird sichergestellt, dass das glockenförmige Element selbst beim Manipulieren der Gebärmutter oder während des gesamten chirurgischen Eingriffs den Muttermund fest umschließt.

In einer weiteren Ausgestaltung der Erfindung sind die Fixierelemente als Stifte ausgebildet.

Diese Maßnahme hat den Vorteil, dass sich die als Stifte ausgebildeten Fixierelemente relativ atraumatisch in das Gewebe im Bereich des Muttermunds hineinstechen, und somit für einen stabilen Sitz des glockenförmigen Elements während des gesamten chirurgischen Eingriffs sorgen.

In einer weiteren Ausgestaltung der Erfindung stehen die Fixierelemente von einer Innenseite des glockenförmigen Elements vor.

Durch derartige Anordnung der Fixierelemente wird sichergestellt, dass der Gebärmuttermund, der in das glockenförmige Element hineinragt, besonders sicher fixiert ist.

In einer weiteren Ausgestaltung der Erfindung ist das glockenförmige Element aus Keramik ausgebildet.

Die Ausbildung des glockenförmigen Elements aus Keramik hat den Vorteil, dass die isolierende Keramik bei chirurgischen Eingriffen mit HF-Instrumenten, die zur Hochfrequenzkoagulation oder zum Hochfrequenzschneiden dienen, hilfreich ist. Die Hochfrequenzkoagulation dient der operativen Zerstörung von Gewebebezirken, zur Blutstillung, zur Abtragung von Gewebebereichen oder zur thermischen Koagulation von Tumoren. Das Hochfrequenzschneiden wird beispielsweise zum Entfernen von Zysten, zum Durchtrennen von Gefäßen oder ähnlichen chirurgischen Eingriffen eingesetzt. Die HF-Instrumente werden oft bei chirurgischen Eingriffen an der Gebärmutter, wie z.B. bei Hysterektomie, eingesetzt.

In einer weiteren bevorzugten Ausgestaltung der Erfindung ist der stabförmige Körper an dem distalen Abschnitt abgebogen.

Diese Maßnahme hat den Vorteil, dass die Bauteile, wie z.B. das glockenförmige Element, wenn diese nach proximal verschoben sind, außerhalb der Längsachse des Abschnitts zum Liegen kommen. Dadurch hat der Operateur freie Sicht längs der Einführrichtung beim Einbringen des distalen Endes des stabförmigen Körpers durch die Vagina in den Gebärmutterhals.

In einer weiteren Ausgestaltung der Erfindung ist das glockenförmige Element über Laufrollen längs des stabförmigen Körpers verschiebbar.

Diese Maßnahme hat den Vorteil, dass das glockenförmige Element verkantfrei geführt längs des stabförmigen Körpers bewegbar ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Fig. 1: eine teilweise geschnittene Seitenansicht eines erfindungsgemäßen Instruments, wobei ein glockenförmiges Element in einer ersten Arretierstelle arretiert ist;
- Fig. 2: eine der Darstellung in Fig. 1 vergleichbare Darstellung, wobei das glockenförmige Element in einer zweiten Arretierstelle arretiert ist;
- Fig. 3: eine vergrößerte geschnittene Seitenansicht des glockenförmigen Elements, bei der das glockenförmige Element kurz vor einer Arretierstelle steht;
- Fig. 4: eine der Darstellung in Fig. 3 vergleichbare Darstellung, wobei das glockenförmige Element in der Arretierstelle arretiert ist;
- Fig. 5: einen Querschnitt durch das erfindungsgemäße Instrument im Bereich des Arretiermechanismus, wobei ein Arretierelement in eine Arretierstelle eingreift;
- Fig. 6: eine der Darstellung in Fig. 5 vergleichbare Darstellung, wobei das Arretierelement von der Arretierstelle außer Eingriff gebracht ist;
- Fig. 7: eine Situation beim Positionieren des medizinischen Instruments in einer Gebärmutter, wobei ein distaler Abschnitt eines stabförmigen Körpers in die Gebärmutter eingeführt ist; und
- Fig. 8: eine der Darstellung in Fig. 7 vergleichbare Darstellung, wobei das glockenfönnige Element den Muttermund umschließt.

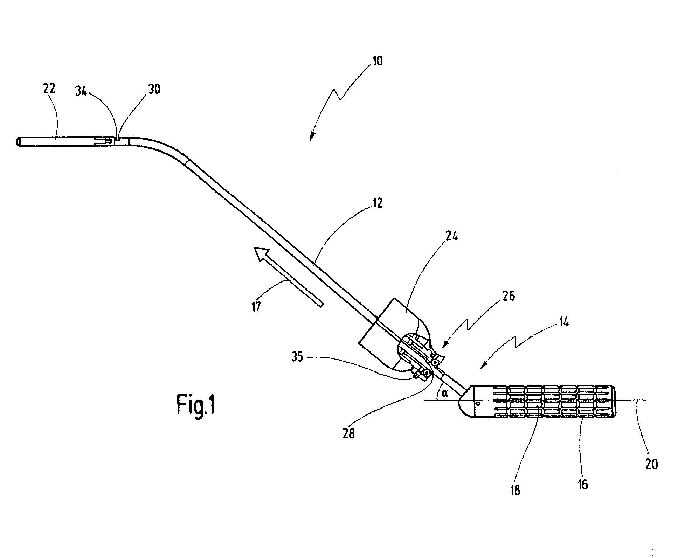

Ein in Fig. 1 dargestelltes medizinisches Instrument zum Manipulieren einer Gebärmutter ist in seiner Gesamtheit mit der Bezugsziffer 10 bezeichnet.

Das erfindungsgemäße medizinische Instrument 10 weist einen stabförmigen Körper 12 auf. An einem proximalen Ende 14 des stabförmigen Körpers 12 ist ein Handgriff 16 angeordnet. In einer Außenseite des Handgriffs 16 ist ein Kerbenmuster 18 eingeschnitten, so dass das medizinische Instrument 1.0 fest und sicher von einer menschlichen Hand im Bereich des Handgriffs 16 ergriffen werden kann.

Der stabförmige Körper 12 ist mit dem Handgriff 16 derart verbunden, dass der stabförmige Körper 12 mit einer Längsachse 20 des Handgriffs 16 einen Winkel α von ca. 45° bildet.

Ein distaler geradliniger Abschnitt 22 des stabförmigen Körpers 12 ist aus der Längsachse des stabförmigen Körpers 12 abgebogen, und zwar so, dass der distale Abschnitt 22 des stabförmigen Körpers 12 und der Handgriff 16 parallel zueinander angeordnet sind.

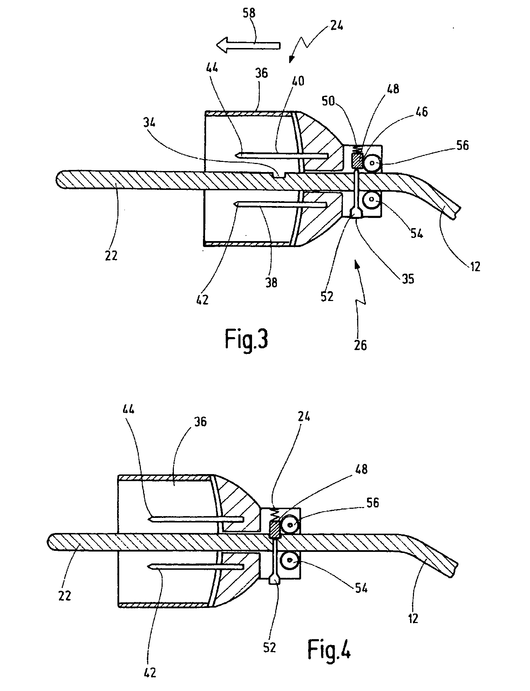

An dem stabförmigen Körper 12 ist ein glockenförmiges Element 24 angeordnet, dessen Ausgestaltung später noch näher in Zusammenhang mit Fig. 3 beschrieben wird. Das glockenförmige Element 24 ist an dem stabförmigen Körper 12 verschiebbar angeordnet. Somit kann das glockenförmige Element 24 längs des stabförmigen Körpers 12 hin und her verschoben werden.

Ferner weist das medizinische Instrument 10 einen Arretiermechanismus 26 auf, mittels dessen das glockenförmige Element 24 in unterschiedlichen axialen Positionen an dem stabförmigen Körper 12 arretierbar ist, wie das später noch näher in Zusammenhang mit Fig. 3 bis 6 beschrieben wird.

In der Darstellung in Fig. 1 ist das glockenförmige Element 24 in einer ersten Arretierstelle 28 arretiert. Die erste Arretierstelle 28 ist an dem proximalen Ende 14 des stabförmigen Körpers 12 angeordnet. Aus dieser Darstellung ist ebenfalls eine zweite Arretierstelle 30 ersichtlich, die an dem distalen Abschnitt 22 des stabförmigen Körpers angeordnet ist. Die beiden Arretierstellen 28, 30 sind als Aussparungen 32, 34 in dem stabförmigen Körper 12 ausgebildet.

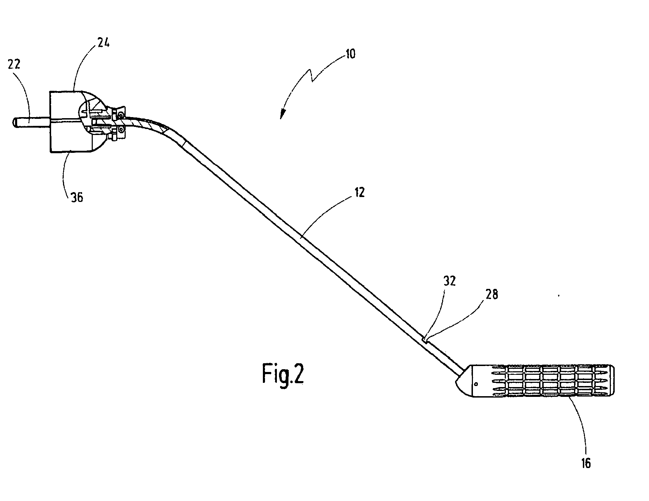

Um das glockenförmige Element 24 von der ersten Arretierstelle 28 in die zweite Arretierstelle 30 zu bringen, muss zunächst ein Betätigungselement 35 zum Entsperren der Arretierung betätigt werden, wie das später noch näher in Zusammenhang mit Fig. 3 bis 6 beschrieben wird. Danach wird das glockenförmige Element 24 längs des stabförmigen Körpers 12 von dem Operateur nach distal (siehe Pfeil 17) verschoben, und zwar so weit, bis das glockenförmige Element 24 mittels des Arretiermechanismus 26 in der zweiten Arretierstelle 30, die an dem distalen Abschnitt 22 des stabförmigen Körpers 12 angeordnet ist, arretiert wird. Solch eine Situation ist in Fig. 2 dargestellt. Der distale Endabschnitt 22 ragt dabei distal über das glockenförmige Element 24 hinaus.

Aus der vergrößerten Darstellung in Fig. 3 und 4 ist die Ausgestaltung des glockenförmigen Elements 24, das in diesem Ausführungsbeispiel aus Keramik ausgebildet ist, ersichtlich.

Das glockenförmige Element 24 weist distalseitig einen zylindrischen Abschnitt 36 auf. Ferner ist das glockenförmige Element 24 an seinem proximalen Ende geschlossen ausgebildet. Dies dient dazu, dass, wenn das glockenförmige Element 24 den Muttermund der Gebärmutter umschließt, keine Gase bzw. Flüssigkeiten aus dem Innenraum der Gebärmutter austreten können.

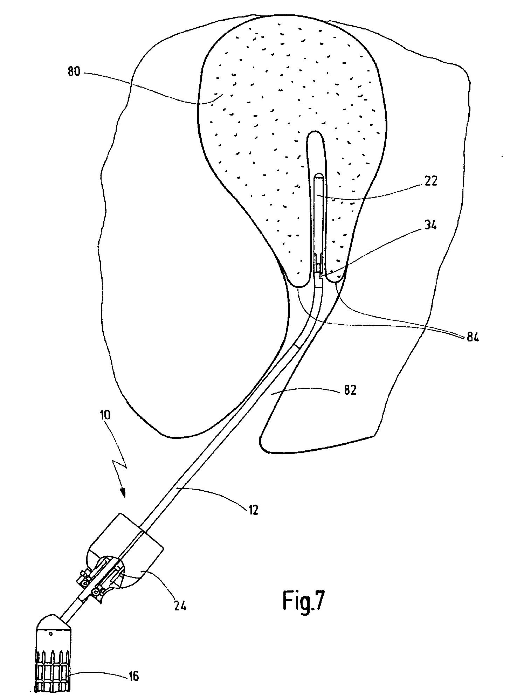

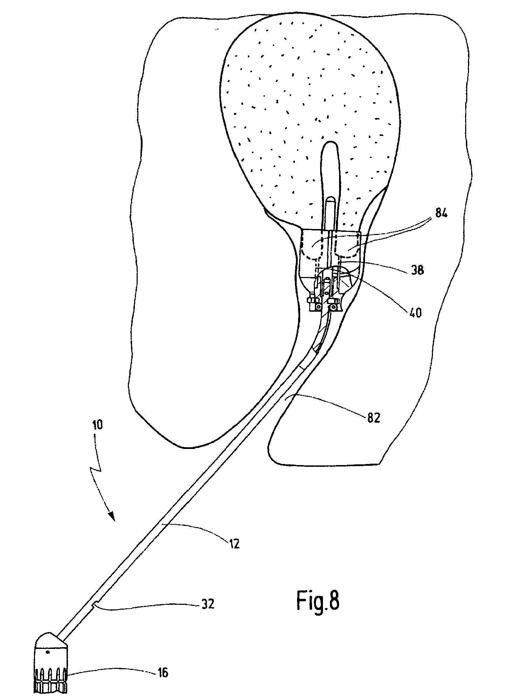

Aus der Darstellung von Fig. 3 und 4 sind zwei von vier nach distal vorspringenden Fixierelementen 38, 40 ersichtlich, die im Inneren des glockenförmigen Elements 24 angeordnet sind. Die Fixierelemente 38, 40 sind in diesem Ausführungsbeispiel als Stifte 42, 44 ausgebildet. Die Fixierelemente 38, 40 dienen zum Fixieren des glockenförmigen Elements 24 an dem Muttermund der Gebärmutter, indem sich die als Stifte 42, 44 ausgebildeten Fixierelemente 38, 40 in das Gewebe im Bereich des Muttermunds hineinstechen, wie das später noch näher in Zusammenhang mit Fig. 7 und 8 beschrieben wird.

Ferner sind in dem glockenförmigen Element 24 zwei gegenüberliegende Laufrollen 54 und 56 vorhanden, zwischen denen der stabförmige Körper 12 liegt. Die Laufrollen 54, 56 dienen zum Führen des glockenförmigen Elements 24 längs des stabförmigen Körpers 12 beim Verschieben.

Aus der vergrößerten, geschnittenen Seitenansicht von Fig. 3 und 4 ist ebenfalls der Arretiermechanismus 26 ersichtlich.

Der Arretiermechanismus 26 weist ein Arretierelement 46 auf, das in diesem Ausführungsbeispiel als federbelastete Raste 48 ausgebildet ist. Die federbelastete Raste 48 ist mittels einer vorgespannten Feder 50 an einer Innenwand des glockenförmigen Elements 24 gelagert.

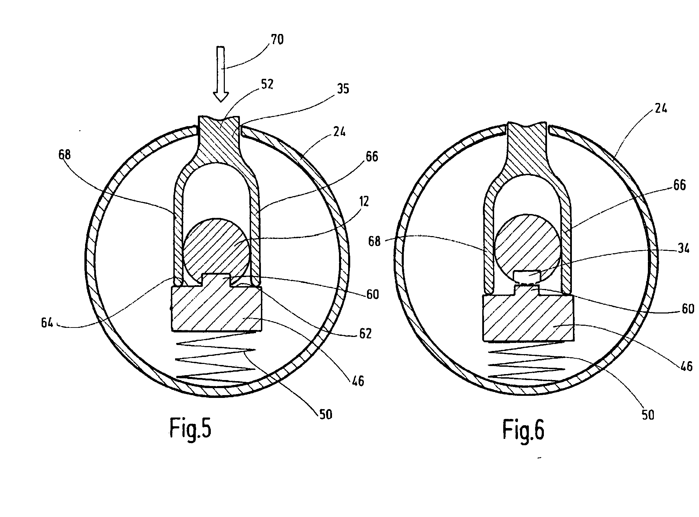

Ferner weist der Arretiermechanismus 26 das oben genannte Betätigungselement 35 zum Entsperren der Raste 48 auf. Das Betätigungselement 35 ist derart ausgebildet, wie das später noch näher in Zusammenhang mit Fig. 5 und 6 beschrieben wird, dass es mit der federbelasteten Raste 48 ständig in Kontakt steht. Das Betätigungselement 35 weist in diesem Ausführungsbeispiel einen Druckknopf 52 auf.

In Fig. 3 ist eine Situation dargestellt, bei der das als federbelastete Raste 48 ausgebildete Arretierelement 46 noch nicht in der zweiten Arretierstelle 30 arretiert ist. In dieser Situation gleitet die federbelastete Raste 48 längs des stabförmigen Körpers 12. Das glockenförmige Element 24 wird von dem Operateur in eine Richtung, die durch einen Pfeil 58 angezeigt ist, so weit verschoben, bis die federbelastete Raste 48 in die als Aussparung 34 ausgebildete zweite Arretierstelle 30 eingreift und in dieser arretiert wird. Solch eine Situation geht aus Fig. 4 hervor.

Bei dem derartig ausgebildeten Arretiermechanismus 26 muss sich der Operateur nicht genau auf das Verschieben des glockenförmigen Elements 24 konzentrieren, weil das Arretierelement 46 selbst einrastend ist. Rastet das Arretierelement 46 in eine der Arretierstellen 28, 30 ein, ist das für den Operateur deutlich hörbar.

Aus dem in Fig. 5 und 6 dargestellten Querschnitt durch das glockenförmige Element 24 ist ersichtlich, dass das als federbelastete Raste 48 ausgebildete Arretierelement 46 an seinem dem stabförmigen Körper 12 zugewandten Ende einen Vorsprung 60 aufweist, der in die als Aussparungen 32, 34 ausgebildete Arretierstellen 28, 30 eingreift. Der Vorsprung 60 springt von zwei Lagerstellen 62, 64 vor. Die Form des Vorsprungs 60 ist der Form der Aussparungen 32, 34 angepasst.

Das dem Arretierelement 46 diametral gegenüberliegende Betätigungselement 35 weist zwei Beine 66, 68 auf, die ständig mit den Lagerstellen 62, 64 des Arretierelements 46 in Kontakt stehen.

In Fig. 5 ist das Arretierelement 46 in der zweiten Arretierstelle 30 arretiert, und das Betätigungselement 35 befindet sich in einem unbetätigten Zustand. Um das Arretierelement 46 mit der zweiten Arretierstelle 30 außer Eingriff zu bringen, wird der Druckknopf 52 von dem Operateur gedrückt. Die Richtung ist durch einen Pfeil 70 angezeigt. Durch das Drücken des Druckknopfs 52 üben die Beine 66, 68 auf die Lagerstellen 62, 64 des Arretierelements 46 eine Kraft aus, die gegen die Federkraft der Feder 50 ausgerichtet ist. Dies reicht aus, um den Vorsprung 60 aus der Aussparung 34 herauszudrücken.

Solch eine Situation ist in Fig. 6 dargestellt. In diesem Zustand kann das glockenförmmige Element 24 längs des stabförmigen Körpers 12 problemlos hin und her verschoben werden. Dabei wird es durch die Laufrollen 54 und 56 geführt.

Der Einsatz des erfindungsgemäßen Instruments 10 soll im Ablauf der Fig. 7 und 8 kurz erläutert werden.

Zuerst wird das glockenförmige Element 24 in der ersten Arretierstelle 28, die sich an dem proximalen Ende 14 des stabförmigen Körpers 12 befindet, arretiert. Dann wird der Handgriff 16 von einer Hand des Operateurs ergriffen, und der distale Abschnitt 22 wird durch die Vagina 82 in die Gebärmutter 80 eingeführt. Solch eine Situation ist in Fig. 7 dargestellt.

Um das glockenförmige Element 24, das in der ersten Arretierstelle 28 arretiert ist, längs des stabförmigen Körpers 12 zu verschieben, wird der Vorsprung 60 des Arretierelements 46 durch Drücken des Druckknopfes 52 mit der Arretierstelle 28 außer Eingriff gebracht, wie das in Fig. 5 und 6 dargestellt ist.

Dann wird das glockenförmige Element 24 von dem Operateur längs des stabförmigen Körpers 12 nach distal verschoben. Er führt das glockenförmige Element 24 in die Vagina 82 so weit hinein, bis er ein Klickgeräusch hört. Dadurch wird sichergestellt, dass das Arretierelement 46 in der zweiten Arretierstelle 30, die an dem distalen Abschnitt 22 angeordnet ist, arretiert ist. In diesem Zustand umschließt der zylindrische Abschnitt 36 des glockenförmigen Elements 24 den Muttermund 84. Beim Umschließen des Muttermunds 84 stechen sich die als Stifte 42, 44 ausgebildeten Fixierelemente 38, 40 in das Gewebe hinein. Dadurch wird das glockenförmige Element 24 in dieser Position fixiert.

Solch eine Situation ist in Fig. 8 dargestellt. In diesem Zustand ist die Gebärmutter 80 dicht durch das glockenförmige Element 24 zur Außenseite hin abgeschlossen.

Je nach Eingriff kann die Vorrichtung 10 samt an dem glockenförmigen Element 24 anhaftendem vom Körper abgetrennten Gewebe des Gebärmutterhalses entfernt werden. Soll nur eine Untersuchung durchgeführt werden, wird das Instrument 10 wieder abgezogen.

## Patentansprüche

1. Medizinisches Instrument zum Manipulieren einer Gebärmutter (80), mit einem stabförmigen Körper (12), dessen distaler Abschnitt (22) durch die Vagina (82) in die Gebärmutter (80) einführbar ist, wobei der stabförmige Körper (12) ein glockenförmiges Element (24) trägt, das längs des stabförmigen Körpers (12) hin und her bewegbar ist, und das glockenförmige Element (24) längs des stabförmigen Körpers (12) verschiebbar ausgebildet ist, **dadurch gekennzeichnet, dass** das glockenförmige Element (24) mittels eines Arretiermechanismus (26) in unterschiedlichen axialen Positionen an dem stabförmigen Körper (12) arretierbar ist.

2. Medizinisches Instrument nach Anspruch 1, **dadurch gekennzeichnet, dass** der Arretiermechanismus (26) ein an dem glockenförmigen Element (24) angeordnetes Arretierelement (46) aufweist, das in den unterschiedlichen axialen Positionen in eine Arretierstelle (28, 30) an dem stabförmigen Körper (12) eingreift.

3. Medizinisches Instrument nach Anspruch 2, **dadurch gekennzeichnet, dass** eine erste Arretierstelle (28) an einem proximalen Ende (14) des stabförmigen Körpers (12) angeordnet ist.

4. Medizinisches Instrument nach Anspruch 3, **dadurch gekennzeichnet, dass** eine zweite Arretierstelle (30) an dem distalen Abschnitt (22) des stabförmigen Körpers (12) angeordnet ist.

5. Medizinisches Instrument nach einem der Ansprüche 2 bis 4, **dadurch gekennzeichnet, dass** das Arretierelement (46) in den Arretierstellen (28, 30) selbst einrastend ist.

6. Medizinisches Instrument nach einem der Ansprüche 2 bis 5, **dadurch gekennzeichnet, dass** die Arretierstellen (28, 30) als Aussparungen (32, 34) in dem stabförmigen Körper (12) ausgebildet sind.

7. Medizinisches Instrument nach einem der Ansprüche 2 bis 6, **dadurch gekennzeichnet, dass** das Arretierelement (46) als federbelastete Raste (48) ausgebildet ist.

8. Medizinisches Instrument nach einem der Ansprüche 2 bis 7, **dadurch gekennzeichnet, dass** der Arretiermechanismus (26) ein Betätigungselement (35) zum Entsperren des Arretierelements (46) aufweist, das an dem glockenförmigen Element (24) angeordnet ist.

9. Medizinisches Instrument nach Anspruch 8, **dadurch gekennzeichnet, dass** das Betätigungselement (35) als Druckknopf (52) ausgebildet ist.

10. Medizinisches Instrument nach einem der Ansprüche 1 bis 9, **dadurch gekennzeichnet, dass** das glockenförmige Element (24) distalseitig einen zylindrischen Abschnitt (36) aufweist.

11. Medizinisches Instrument nach einem der Ansprüche 1 bis 10, **dadurch gekennzeichnet, dass** das glockenförmige Element (24) an seinem proximalen Ende geschlossen ist.

12. Medizinisches Instrument nach einem der Ansprüche 1 bis 11, **dadurch gekennzeichnet, dass** an dem glockenförmigen Element (24) nach distal vorspringende Fixierelemente (38, 40) angeordnet sind.

13. Medizinisches Instrument nach Anspruch 12, **dadurch gekennzeichnet, dass** die Fixierelemente (38, 40) als Stifte (42, 44) ausgebildet sind.

14. Medizinisches Instrument nach Anspruch 12 oder 13, **dadurch gekennzeichnet, dass** die Fixierelemente (38, 40) von einer Innenseite des glockenförmigen Elements (24) vorstehen.

15. Medizinisches Instrument nach einem der Ansprüche 1 bis 14, **dadurch gekennzeichnet, dass** das glockenförmige Element (24) aus Keramik ausgebildet ist.

16. Medizinisches Instrument nach einem der Ansprüche 1 bis 15, **dadurch gekennzeichnet, dass** der stabförmige Körper (12) an dem distalen Abschnitt (22) gebogen ausgebildet ist.

17. Medizinisches Instrument nach einem der Ansprüche 1 bis 16, **dadurch gekennzeichnet, dass** das glockenförmige Element (24) über Laufrollen (54, 56) längs des stabförmigen Körpers (12) verschiebbar ist.

## Claims

1. Medical instrument for manipulation of an uterus (80), with a rod-shaped body (12) whose distal portion (22) can be inserted through the vagina (82) into the uterus (80), said rod-shaped body (12) carrying a bell-shaped element (24) that can be moved to and fro along the rod-shaped body (12), and wherein the bell-shaped element (24) is designed to be displaceable along the rod-shaped body (12), **characterized in that** the bell-shaped element (24) can be locked by means of a locking mechanism (26) in different axial positions on the rod-shaped body (12).

2. Medical instrument of Claim 1, **characterized in that** the locking mechanism (26) has a locking element (26) which is arranged on the bell-shaped element (24) and which, in the different axial positions, engages in a locking site (28, 30) on the rod-shaped body (12).

3. Medical instrument of Claim 2, **characterized in that** a first locking site (28) is arranged at a proximal end (14) of the rod-shaped body (12).

4. Medical instrument of Claim 3, **characterized in that** a second locking site (30) is arranged at a distal portion (22) of the rod-shaped body (12).

5. Medical instrument of anyone of Claims 2 through 4, **characterized in that** the locking element (46) locks automatically into the locking sites (28, 30).

6. Medical instrument of anyone of Claims 2 through 5, **characterized in that** the locking sites (28, 30) are designed as recesses (32, 34) in the rod-shaped body (12).

7. Medical instrument of anyone of Claims 2 through 6, **characterized in that** the locking element (46) is designed as a spring-loaded catch (48).

8. Medical instrument of anyone of Claims 2 through 7, **characterized in that** the locking mechanism (26) has an actuating element (35) for releasing the locking element (46) arranged on the bell-shaped element (24).

9. Medical instrument of Claim 8, **characterized in that** the actuating element (35) is designed as a push-button (52).

10. Medical instrument of anyone of Claims 1 through 9, **characterized in that** the bell-shaped element (24) has a cylindrical portion (36) at its distal end.

11. Medical instrument of anyone of Claims 1 through 10, **characterized in that** the bell-shaped element (24) is closed at its proximal end.

12. Medical instrument of anyone of Claims 1 through 11, **characterized in that** distally projecting fixing elements (38, 40) are arranged on the bell-shaped element (24).

13. Medical instrument of Claim 12, **characterized in that** the fixing elements (38, 40) are designed as pins (42, 44).

14. Medical instrument of Claims 12 or 13, **characterized in that** the fixing elements (38, 40) protrude from an inner face of the bell-shaped element (24).

15. Medical instrument of anyone of Claims 1 through 14, **characterized in that** the bell-shaped element (24) is made of ceramic.

16. Medical instrument of anyone of Claims 1 through 15, **characterized in that** the rod-shaped body (12) is bent at the distal portion (22).

17. Medical instrument of anyone of Claims 1 through 16, **characterized in that** the bell-shaped element (24) is displaceable along the rod-shaped body (12) on rollers (54, 56).

## Revendications

1. Instrument médical destiné à la manipulation d'un utérus (80), comportant un corps en forme de tige (12) dont le segment distal (22) peut être introduit dans l'utérus (80) par le vagin (82), le corps en forme de tige (12) portant un élément en forme de cloche (24) qui peut être déplacé d'avant en arrière le long du corps en forme de tige (12), l'élément en forme de cloche (24) étant conçu ou réalisé pour coulisser le long du corps en forme de tige (12), **caractérisé en ce que** l'élément en forme de cloche (24) peut être arrêté au moyen d'un mécanisme d'arrêt (26) dans différentes positions axiales sur le corps en forme de tige (12).

2. Instrument médical selon la revendication 1, **caractérisé en ce que** le mécanisme d'arrêt (26) présente un élément d'arrêt (46) disposé sur l'élément en forme de cloche (24) et qui s'insère dans les différentes positions axiales dans un emplacement d'arrêt (28, 30) au niveau du corps en forme de tige (12).

3. Instrument médical selon la revendication 2, **caractérisé en ce qu'**une première position d'arrêt (28) est disposée au niveau d'une extrémité proximale (14) du corps en forme de tige (12).

4. Instrument médical selon la revendication 3, **caractérisé en ce qu'**une seconde position d'arrêt (30) est disposée au niveau du segment distal (22) du corps en forme de tige (12).

5. Instrument médical selon l'une des revendications 2 à 4, **caractérisé en ce que** l'élément d'arrêt (46) peut s'encliqueter automatiquement dans les emplacements d'arrêt (28, 30).

6. Instrument médical selon l'une des revendications 2 à 5, **caractérisé en ce que** les emplacements d'arrêt (28, 30) sont conçus en tant qu'évidements (32, 34) dans le corps en forme de tige (12).

7. Instrument médical selon l'une des revendications 2 à 6, **caractérisé en ce que** l'élément d'arrêt (46) est conçu en tant que cran chargé par ressort (48).

8. Instrument médical selon l'une des revendications 2 à 7, **caractérisé en ce que** le mécanisme d'arrêt (26) présente un élément d'actionnement (35) destiné à débloquer l'élément d'arrêt (46) et qui est disposé au niveau de l'élément en forme de cloche (24).

9. Instrument médical selon la revendication 8, **caractérisé en ce que** l'élément d'actionnement (35) est réalisé en tant que bouton-poussoir (52).

10. Instrument médical selon l'une des revendications 1 à 9, **caractérisé en ce que** l'élément en forme de cloche (24) présente côté distal un segment ou portion cylindrique.

11. Instrument médical selon l'une des revendications 1 à 10, **caractérisé en ce que** l'élément en forme de cloche (24) est fermé au niveau de son extrémité proximale.

12. Instrument médical selon l'une des revendications 1 à 11, **caractérisé en ce qu'**au niveau de l'élément en forme de cloche (24) sont disposés des éléments de fixation faisant saillie vers le côté distal (38, 40).

13. Instrument médical selon la revendication 12, **caractérisé en ce que** les éléments de fixation (38, 40) sont réalisés sous la forme de goupilles (42, 44).

14. Instrument médical selon la revendication 12 ou 13, **caractérisé en ce que** les éléments de fixation (38, 40) font saillie depuis un côté interne de l'élément en forme de cloche (24).

15. Instrument médical selon l'une des revendications 1 à 14, **caractérisé en ce que** l'élément en forme de cloche (24) est réalisé en céramique.

16. Instrument médical selon l'une des revendications 1 à 15, **caractérisé en ce que** le corps en forme de tige (12) est réalisé de façon courbée au niveau du segment distal (22).

17. Instrument médical selon l'une des revendications 1 à 16, **caractérisé en ce que** l'élément en forme de cloche (24) peut être déplacé sur des galets (54, 56) le long du corps en forme de tige (12).
